# EUROPEAN PATENT SPECIFICATION

(11) **EP 3 937 840 B1**
(45) Date of publication and mention of the grant of the patent: **29.05.2024**
(21) Application number: 20719669.2
(22) Date of filing: 12.03.2020
(51) Int. Cl.: A61C 9/00, A61C 19/04

(54) **GENERATION OF TRANSFORMATION MATRICES ASSOCIATED WITH UPPER AND LOWER DENTAL ARCHES**
ERZEUGUNG VON TRANSFORMATIONSMATRIZEN IM ZUSAMMENHANG MIT OBEREN UND UNTEREN ZAHNBÖGEN
GÉNÉRATION DE MATRICES DE TRANSFORMATION ASSOCIÉES À DES ARCADES DENTAIRES SUPÉRIEURES ET INFÉRIEURES

(30) Priority: 12.03.2019 FI 20195182
(43) Date of publication of application: 19.01.2022
(73) Proprietor: Planmeca OY, 00880 Helsinki (FI)
(72) Inventor: SALONEN, Ville, 00880 Helsinki (FI); NAUKKARINEN, Hanna, 00880 Helsinki (FI); VALLINKOSKI, Irene, 00880 Helsinki (FI); SYRJÄ, Ismo, 00880 Helsinki (FI)
(86) International application number: PCT/FI2020/050157
(87) International publication number: WO 2020/183066

(56) References cited:
- EP-A1- 1 759 353
- EP-B1- 1 759 353
- US-A1- 2002 064 746
- US-A1- 2008 176 182
- US-A1- 2016 163 115

## Description

### FIELD OF THE INVENTION

The invention relates to the field of medical imaging. In particular, the invention relates to the generation of transformation matrices associated with upper and lower dental arches to be used in orthognathic operations.

### BACKGROUND

In orthognathic operation planning a target occlusion can be obtained by an orthodontist by manually moving upper and lower plaster casts to target occlusion before a virtual surgery planning. The plaster casts are needed in creation of a target occlusion. The target occlusion model may then be digitized using a desktop scanner, and virtual surgical jaw movements are performed with appropriate software by a surgeon using the occlusion model to find correct transformation matrices of upper and lower impressions.

In order to obtain the plaster casts, a patient needs to be involved in an uncomfortable impression taking process. Further, the process requires time also by a technician involved in the process. The plaster casts may also break easily. Further, the impression taking process may not be accurate and the plaster casts need to be stored for a certain period of time.

Therefore, there is a need for a solution that would enable a more efficient and accurate process for obtaining the transformation matrices to be used in orthognathic operations.

US2008/176182 discloses a system and method for determining condyle displacement during jaw articulation includes a physical model with corresponding reference points, wherein the physical model is positioned and scanned to obtain positional data representing a first and second bite position and this positional data is used to generate a transformation matrix.

### SUMMARY

The invention is as defined in the appended claims. T According to at least some of the aspects, a solution is provided that avoids the traditional impression-taking and plaster casts while still allowing the possibility to determine the target occlusion manually and obtain haptic feedback of the target occlusion. A digital impression of upper and lower dental arches, or a first digital impression of said upper dental arch and a second digital impression of said lower dental arch of a patient may be obtained, for example, based on scanning data of said upper and lower dental arches, and the digital impression(s) can then be used to generate separate physical three-dimensional models of the upper and lower dental arches. After creating a desired target occlusion with the physical three-dimensional models of the upper and lower dental arches, a scan may be performed based on the desired target occlusion. The transformation matrices needed in orthognathic operations can then be determined based on the digital impression(s) and data from the scan.

According to a first aspect, there is provided a method for generating transformation matrices associated with upper and lower dental arches. The method comprises obtaining a digital impression of said upper and lower dental arches, or a first digital impression of said upper dental arch and a second digital impression of said lower dental arch; generating a digital three-dimensional model comprising said upper and lower dental arches, or a first digital three-dimensional model comprising said upper dental arch and a second digital three-dimensional model comprising said lower dental arch based on said digital impression(s); outputting said digital three-dimensional model(s) to produce printed separate physical three-dimensional models of said upper and lower dental arches; obtaining scanning data associated with a desired target occlusion, said desired target occlusion being obtained based on a desired mutual position of said printed physical three-dimensional models of said upper and lower dental arches, the scanning data comprising only a portion of a complete buccal scan of the printed physical three-dimensional models of the upper and lower dental arches; and determining said transformation matrices of said upper and lower dental arches to said desired target occlusion based on said scanning data and said digital impression(s).

According to a second aspect, there is provided a method for generating transformation matrices associated with upper and lower dental arches. The method comprises obtaining a digital impression of said upper and lower dental arches, or a first digital impression of said upper dental arch and a second digital impression of said lower dental arch, said digital impression(s) being based on printed physical three-dimensional models of said upper and lower dental arches, the printed physical three-dimensional models being prepared based on digital dental data; obtaining scanning data associated with a desired target occlusion, said desired target occlusion being obtained based on a desired mutual position of said printed physical three-dimensional models of said upper and lower dental arches, the scanning data comprising only a portion of a complete buccal scan of the printed physical three-dimensional models of the upper and lower dental arches; and determining said transformation matrices of said upper and lower dental arches to said desired target occlusion based on said scanning data and said digital impression(s).

In an embodiment, data associated with said digital impression(s) and/or scanning data are obtained from an intraoral scanning device.

According to a third aspect, there is provided a system for generating transformation matrices associated with upper and lower dental arches. The system comprises at least one processing unit and at least one memory connected to said at least one processing unit. Said at least one memory stores program instructions that, when executed by said at least one processing unit, cause said system to obtain digital impression of said upper and lower dental arches, or a first digital impression of said upper dental arch and a second digital impression of said lower dental arch; generate a digital three-dimensional model comprising said upper and lower dental arches, or a first digital three-dimensional model comprising said upper dental arch and a second digital three-dimensional model comprising said lower dental arch based on said digital impression(s); output said digital three-dimensional model(s) to produce separate printed physical three-dimensional models of said upper and lower dental arches; obtain scanning data associated with a desired target occlusion, said desired target occlusion being obtained based on a desired mutual position of said physical three-dimensional models of said upper and lower dental arches, the scanning data comprising only a portion of a complete buccal scan of the printed physical three-dimensional models of the upper and lower dental arches; and determine said transformation matrices of said upper and lower dental arches to said desired target occlusion based on said scanning data and said digital impression(s).

According to a fourth aspect, there is provided a system for generating transformation matrices associated with upper and lower dental arches. The system comprises at least one processing unit and at least one memory connected to said at least one processing unit. Said at least one memory stores program instructions that, when executed by said at least one processing unit, cause said system to obtain a digital impression of said upper and lower dental arches, or a first digital impression of said upper dental arch and a second digital impression of said lower dental arch, said digital impression(s) being based on printed physical three-dimensional models of said upper and lower dental arches, the printed physical three-dimensional models being prepared based on digital dental data; obtain scanning data associated with a desired target occlusion, said desired target occlusion being obtained based on a desired mutual position of said printed physical three-dimensional models of said upper and lower dental arches, the scanning data comprising only a portion of a complete buccal scan of the printed physical three-dimensional models of the upper and lower dental arches; and determine said transformation matrices of said upper and lower dental arches to said desired target occlusion based on said scanning data and said digital impression(s).

In an embodiment, the system further comprises an intraoral scanning device configured to obtain the data associated with said digital impression(s) and/or scanning data.

According to a fifth aspect, there is provided a computer program comprising program code, which when executed by at least one processing unit, causes said at least one processing unit to perform the method of the first or second aspect.

According to a sixth aspect, there is provided a computer readable medium comprising program code, which when executed by at least one processor, causes said at least one processor to perform the method of any the first or second aspect.

According to a seventh aspect, there is provided a system for generating transformation matrices associated with upper and lower dental arches. The system comprises means for obtaining digital impression of said upper and lower dental arches, or a first digital impression of said upper dental arch and a second digital impression of said lower dental arch; means for generating a digital three-dimensional model comprising said upper and lower dental arches, or a first digital three-dimensional model comprising said upper dental arch and a second digital three-dimensional model comprising said lower dental arch based on said digital impression(s); means for outputting said digital three-dimensional model(s) to produce separate printed physical three-dimensional models of said upper and lower dental arches; means for obtaining scanning data associated with a desired target occlusion, said desired target occlusion being obtained based on a desired mutual position of the printed physical three-dimensional models of said upper and lower dental arches, the scanning data comprising only a portion of a complete buccal scan of the printed physical three-dimensional models of the upper and lower dental arches; and means for determining said transformation matrices of said upper and lower dental arches to said desired target occlusion based on said scanning data and said digital impression(s).

According to an eight aspect, there is provided a system for generating transformation matrices associated with upper and lower dental arches. The system comprises means for obtaining a digital impression of said upper and lower dental arches, or a first digital impression of said upper dental arch and a second digital impression of said lower dental arch, said digital impression(s) being based on printed physical three-dimensional models of said upper and lower dental arches, the printed physical three-dimensional models being prepared based on digital dental data; means for obtaining scanning data associated with a desired target occlusion, said desired target occlusion being obtained based on a desired mutual position of said printed physical three-dimensional models of said upper and lower dental arches, the scanning data comprising only a portion of a complete buccal scan of the printed physical three-dimensional models of the upper and lower dental arches; and means for determining said transformation matrices of said upper and lower dental arches to said desired target occlusion based on said scanning data and said digital impression(s).

### BRIEF DESCRIPTION OF THE DRAWINGS

The accompanying drawings, which are included to provide a further understanding of the invention and constitute a part of this specification, illustrate embodiments of the invention and together with the description help to explain the principles of the invention. In the drawings:
**FIG. 1A** illustrates a flow diagram of a method generating transformation matrices associated with upper and lower dental arches according to an embodiment.
**FIG. 1B** illustrates another flow diagram of a method generating transformation matrices associated with upper and lower dental arches according to an embodiment.
**FIG. 2A** illustrates an example of physical three-dimensional models of the upper and lower dental arches.
**FIG. 2B** illustrates an example of a target occlusion created with the physical three-dimensional models.
**FIG. 2C** illustrates the process of obtaining buccal scanning data associated with the desired target occlusion based on the physical three-dimensional models.
**FIG. 3** illustrates a system diagram depicting an exemplary system including a variety of optional hardware and software components.

### DETAILED DESCRIPTION

Reference will now be made in detail to the embodiments of the present invention, examples of which are illustrated in the accompanying drawings.

The following description provides a solution that enables efficient and more accurate process for obtaining the transformation matrices to be used in orthognathic operations. Instead of preparing plaster casts of upper and lower dental arches, the solution uses a digital impression or digital impressions obtained, for example, based on scanning data of the upper and lower dental arches of a patient's mouth. The scanning data may be provided, for example, by an intraoral scanner. The digital impression(s) may then be used to prepare separate physical three-dimensional models of the upper and lower dental arches, and the physical three-dimensional models can then be used to create a desired target occlusion. Based on the desired target occlusion, it is then possible to determine the needed transformation matrices of the upper and lower dental arches to the desired target occlusion.

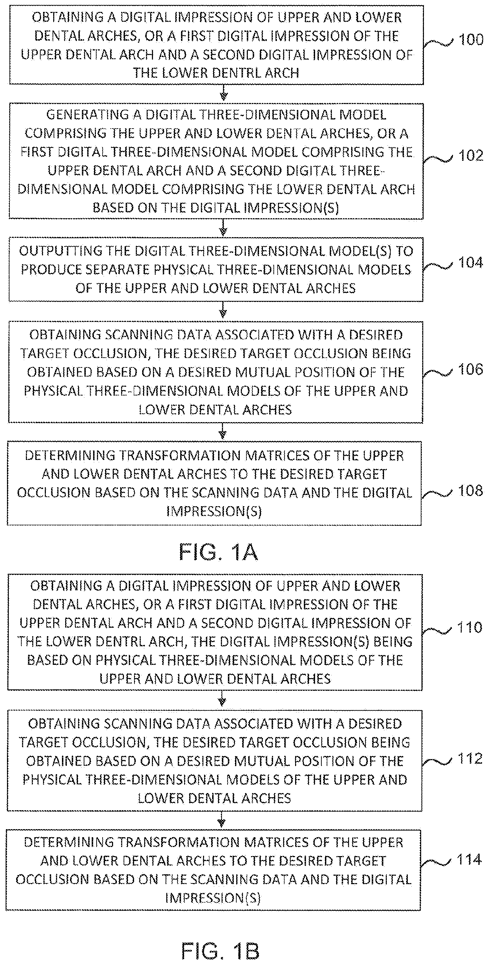

FIG. 1A illustrates a flow diagram of a method generating transformation matrices associated with upper and lower dental arches according to an embodiment.

At 100 a three-dimensional digital impression of the upper and lower dental arches, or a first digital impression of the upper dental arch and a second digital impression of the lower dental arch is/are obtained, for example, based on scanning data of the upper and lower dental arches. In another embodiment, multiple digital impressions altogether comprising the upper and lower dental arches may be obtained. This means that multiple digital impressions together may represent the upper and lower dental arches. The scanning data may refer, for example, to data produced with and received from an intraoral scanner. Thus, it is possible to generate the three-dimensional digital impression(s) of the upper and lower dental arches based on the data received from the intraoral scanner. In other embodiments, any other technology enabling to provide the three-dimensional digital impression, for example, three-dimensional X-ray technology, may be used. Further, in an embodiment, a three-dimensional digital impression of at least part of the upper and lower dental arches, or a first digital impression of at least part of the upper dental arch and a second digital impression of at least part of the lower dental arch may be obtained instead of complete upper and lower dental arches.

At 102 a digital three-dimensional model comprising the upper and lower dental arches, or a first digital three-dimensional model comprising the upper dental arch and a second digital three-dimensional model comprising the lower dental arch is/are generated based on the three-dimensional digital impression(s). The digital three-dimensional model accurately reproduces the upper and lower dental arches. The digital three-dimensional model may be represented by one or more files that can later be used in producing separate physical three-dimensional models of the upper and lower dental arches. In another embodiment, instead of a single digital three-dimensional model, separate digital three-dimensional models may be prepared for the upper dental arch and the lower dental arch.

At 104 the digital three-dimensional model(s) is/are output to produce separate physical three-dimensional models of the upper and lower dental arches. The outputting may refer, for example, to transferring a file or files comprising the digital three-dimensional model or models via a data interface to enable later production of the physical three-dimensional models of the upper and lower dental arches. In another example, the file may be transferred directly to a computer-aided manufacturing (CAM) device, for example, to a 3D printing device, to obtain the physical three-dimensional models of the upper and lower dental arches.

At 106 scanning data associated with a desired target occlusion is obtained, the desired target occlusion being obtained based on a desired mutual position of the physical three-dimensional models of the upper and lower dental arches. The term "scan" may generally refer to a scan where lower and upper dental arches are in an occlusion state. Scanning data may comprise data only about gums and in the occlusion state. In another embodiment, the scanning data may comprise data about both gums and teeth in the occlusion state. Yet in another embodiment, the scanning data may comprise data teeth in the occlusion state. The scanning data may originate, for example, from an intraoral scanner providing digital imaging data. The physical three-dimensional models of the upper and lower dental arches may have been positioned to the desired target occlusion, and the intraoral scanner may have been used to perform the scanning. In an embodiment, the scanning data comprises only a portion or portions of a complete buccal scan of the physical three-dimensional models of the upper and lower dental arches. As an example, the scanning data may comprise data about a molar area of the teeth, either only from one side or from both sides. In another embodiment, the scanning data may comprise a complete buccal scan of the physical three-dimensional models of the upper and lower dental arches. Further, in an embodiment, the scanning data may originate from a lingual side of the physical three-dimensional models of the upper and lower dental arches.

At 108 the transformation matrices of the upper and lower dental arches to the desired target occlusion are determined based on the scanning data and the digital impression(s). As the digital impression or digital impressions is/are used to generate the physical three-dimensional models of the upper and lower dental arches, the scanning data very accurately corresponds with the digital three-dimensional model of the upper and lower dental arches. This may mean that it is sufficient to scan only a portion of the upper and lower dental arches in the desired target occlusion based on which it is possible to determine the transformation matrices. For example, if the scanning data comprises data only for a limited amount of teeth, for example, 1-3 teeth from both the upper dental arch and the lower dental arch in the target occlusion, it is still possible to determine the transformation matrices. The remaining parts of the desired target occlusion may be determined based on the digital impressions, and thus all the necessary transformation matrices can be calculated. The limited amount of teeth may comprises a tooth or teeth, for example, from at least one of buccal, labial or lingual parts. As the scanning data very accurately corresponds with the digital three-dimensional model of the upper and lower dental arches, this also may mean that the scanning data may comprise only a small portion of a complete scan or buccal scan of the upper and lower dental arches, and it is possible to determine the desired target occlusion based on the small portion of the complete scan.

FIG. 1B illustrates another flow diagram of a method generating transformation matrices associated with upper and lower dental arches according to an embodiment.

At 110 a digital impression of the upper and lower dental arches, or a first digital impression of the upper dental arch and a second digital impression of the lower dental arch is/are obtained, the digital impression(s) being based on physical three-dimensional models of the upper and lower dental arches. The physical three-dimensional models may have been prepared earlier based on dental data associated with a patient. The physical three-dimensional models may have been produced based on data produced, for example, with an intraoral scanner.

At 112 scanning data associated with a desired target occlusion is obtained, the desired target occlusion being obtained based on a desired mutual position of the physical three-dimensional models of the upper and lower dental arches. The term "scan" may generally refer to a scan where lower and upper dental arches are in an occlusion state. The scanning data may comprise data only about gums and in the occlusion state. In another embodiment, the scanning data may comprise data about both gums and teeth in the occlusion state. Yet in another embodiment, the scanning data may comprise data teeth in the occlusion state. The scanning data may originate, for example, from an intraoral scanner providing digital imaging data. The physical three-dimensional models of the upper and lower dental arches may have been positioned to the desired target occlusion, and the intraoral scanner may have been used to perform the scanning. In an embodiment, the scanning data comprises only a portion or portions of a complete buccal scan of the physical three-dimensional models of the upper and lower dental arches. As an example, the scanning data may comprise data about a molar area of the teeth, either only from one side or from both sides. In another embodiment, the scanning data may comprise a complete scan or a buccal scan of the physical three-dimensional models of the upper and lower dental arches. Further, in an embodiment, the scanning data may originate from a lingual side of the physical three-dimensional models of the upper and lower dental arches.

At 114 the transformation matrices of the upper and lower dental arches to the desired target occlusion are determined based on the scanning data and the digital impression(s).

The solution illustrated in FIG. 1B provides a solution in which the at least one digital impression and the scanning data are obtained based on the same physical three-dimensional models of the upper and lower dental arches.

An advantage of the solution discussed above is the possibility to avoid the traditional impression-taking and plaster casts while still allowing the possibility to determine the target occlusion manually and obtain haptic feedback of the target occlusion. Further, when using the original digital impressions obtained by scanning the dental arches from a patient's mouth, it is sufficient to subsequently scan only the desired target occlusion from the physical three-dimensional models of the upper and lower dental arches or only part of the desired target occlusion.

The steps 100-114 discussed above may be implemented as a computer-implemented method by using one or more software programs executable in a data processing device, for example, a computer.

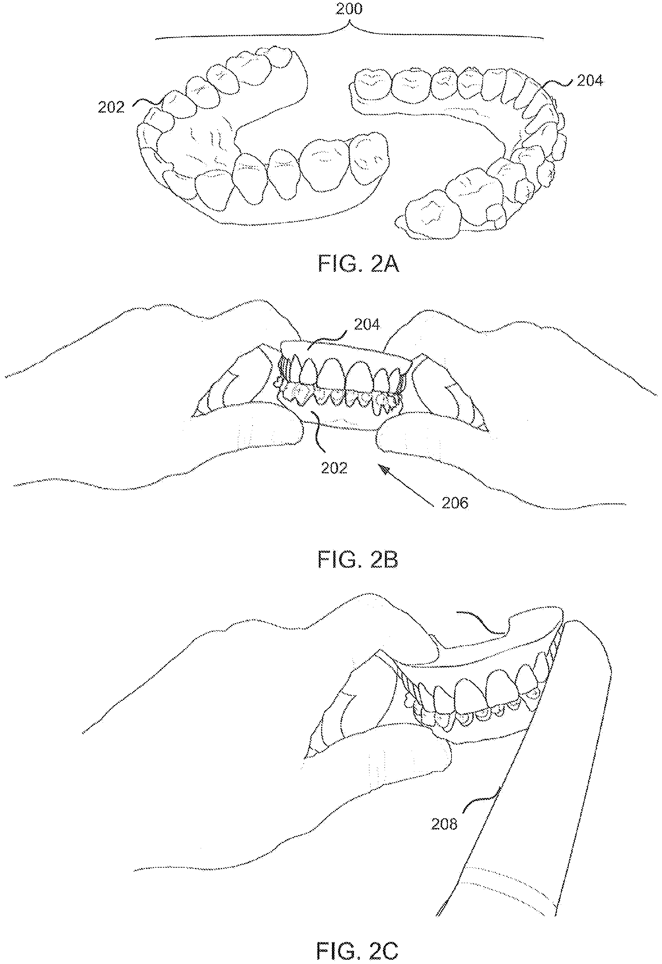

FIG. 2A illustrates an example of physical three-dimensional models 200 of the upper and lower dental arches 204, 202. The lower dental arch 202 and the upper dental arch may be printed, for example, with a 3D printer based on digital impressions scanned using, for example, an intraoral scanner from a patient's mouth. When the physical three-dimensional models 200 are produced based on the digital impressions, the physical three-dimensional models 200 are much more accurate copies of the upper and lower dental arches than copies obtained by an uncomfortable impression taking process producing plaster casts.

FIG. 2B illustrates an example of a desired target occlusion 206 created with the physical three-dimensional models 200. The desired target occlusion 206 may be reached by manually moving the lower and upper dental arches 202, 204 to a desired position with respect to each other.

FIG. 2C illustrates the process of obtaining scanning data or buccal scanning data associated with the desired target occlusion 206 based on the physical three-dimensional models 200. The buccal scanning data associated with the desired target occlusion 206 may be obtained by using an intraoral scanner 208. An orthodontist may use an intraoral scanner 208 to scan at least a portion of a complete buccal scan of the physical three-dimensional models 200 of the upper and lower dental arches. And more generally, in an example embodiment, it is sufficient to scan only a portion of the upper and lower dental arches in the desired target occlusion based on which it is possible to determine the transformation matrices. For example, if the scanning data comprises data only for a limited amount of teeth, for example, 1-3 teeth from both the upper dental arch and the lower dental arch in the target occlusion, it is still possible to determine the transformation matrices. The remaining parts of the desired target occlusion may be determined based on the digital impressions, and thus all the necessary transformation matrices can be calculated. The limited amount of teeth may comprises a tooth or teeth, for example, from at least one of buccal, labial or lingual parts. Thus, as the physical three-dimensional models 200 have been produced based on digital impressions of the upper and lower dental arches of the patient, it may be sufficient to perform the scan only for a small portion of the physical three-dimensional models 200 in the desired target occlusion 206. Further, the use of the intraoral scanner 208 enables an efficient and easy way to obtain data about upper and lower dental arches in the desired target occlusion 206.

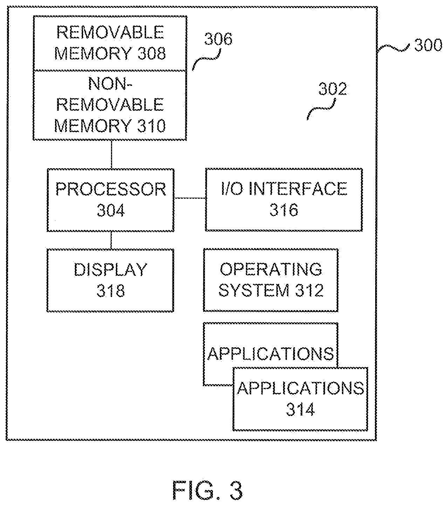

FIG. 3 illustrates a system or apparatus diagram depicting an exemplary system 300 including a variety of optional hardware and software components, shown generally at 302. Any components 302 in the system 300 can communicate with any other component, although not all connections are shown, for ease of illustration. The system 300 can be any of a variety of computing devices (for example, a computer, a laptop computer, a cloud-based server etc.) and can allow two-way communications with one or more communications networks, such as the Internet.

The illustrated system 300 can include one or more controllers or processors 304 (e.g., signal processor, microprocessor, ASIC, or other control and processing logic circuitry) for performing such tasks as signal coding, data processing, input/output processing, power control, and/or other functions. An operating system 312 can control the allocation and usage of the components 302 and support for one or more application programs 314. The application programs can include common computing applications (e.g., server software), or any other computing application.

The illustrated system 300 can include a memory 306. The memory 306 can include non-removable memory 310 and/or removable memory 308. The non-removable memory 310 can include RAM, ROM, flash memory, a hard disk, or other well-known memory storage technologies. The removable memory 308 can include flash memory or other well-known memory storage technologies. The memory 306 can be used for storing data and/or code for running the operating system 312 and the applications 314. Example data can include text, images, sound files, video data, or other data sets to be sent to and/or received from one or more network servers or other devices via one or more wired or wireless networks. The system 300 can further include a display 318 and at least one input/output interface 316, which can be a USB port, IEEE 1494 (FireWire) port, and/or RS-242 port etc.

The illustrated components 302 are not required or all-inclusive, as any components can be deleted and other components can be added.

The system 300 may be configured to implement the various features, examples and embodiments illustrated in FIGS. 1A and 1B partially or completely. The functionality described herein can be performed, at least in part, by one or more computer program product components such as software components. According to an example, the processor 304 may be configured by the program code which when executed performs the examples and embodiments of the operations and functionality described. Alternatively, or in addition, the functionality described herein can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), Graphics Processing Units (GPUs).

Further, one or more of the disclosed elements or components 302 of the system 300 may constitute means for obtaining a digital impression of the upper and lower dental arches, or a first digital impression of the upper dental arch and a second digital impression of the lower dental arch; means for generating a digital three-dimensional model comprising the upper and lower dental arches, or a first digital three-dimensional model comprising the upper dental arch and a second digital three-dimensional model comprising the lower dental arch based on the digital impression(s); means for outputting the digital three-dimensional model(s) to produce separate physical three-dimensional models of the upper and lower dental arches; means for obtaining scanning data associated with a desired target occlusion, the desired target occlusion being obtained based on a desired mutual position of the physical three-dimensional models of the upper and lower dental arches; and means for determining the transformation matrices of the upper and lower dental arches to the desired target occlusion based on the scanning data and the digital impression(s).

Further, one or more of the disclosed elements or components 302 of the system 300 may constitute means for obtaining a digital impression of the upper and lower dental arches, or a first digital impression of the upper dental arch and a second digital impression of the lower dental arch, the digital impression(s) being based on physical three-dimensional models of the upper and lower dental arches; means for obtaining scanning data associated with a desired target occlusion, the desired target occlusion being obtained based on a desired mutual position of the physical three-dimensional models of the upper and lower dental arches; and means for determining the transformation matrices of the upper and lower dental arches to the desired target occlusion based on the scanning data and the digital impression(s).

Further, in one embodiment, the processor 304 may be configured to perform the method steps discussed in FIGS. 1A and/or FIG. 1B.

Example embodiments may be implemented in software, hardware, application logic or a combination of software, hardware and application logic. The example embodiments can store information relating to various methods described herein. This information can be stored in one or more memories, such as a hard disk, optical disk, magneto-optical disk, RAM, and the like. One or more databases can store the information used to implement the example embodiments. The databases can be organized using data structures (e.g., records, tables, arrays, fields, graphs, trees, lists, and the like) included in one or more memories or storage devices listed herein. The methods described with respect to the example embodiments can include appropriate data structures for storing data collected and/or generated by the methods of the devices and subsystems of the example embodiments in one or more databases.

All or a portion of the example embodiments can be conveniently implemented using one or more general purpose processors, microprocessors, digital signal processors, micro-controllers, and the like, programmed according to the teachings of the example embodiments, as will be appreciated by those skilled in the computer and/or software art(s). Appropriate software can be readily prepared by programmers of ordinary skill based on the teachings of the example embodiments, as will be appreciated by those skilled in the software art. In addition, the example embodiments can be implemented by the preparation of application-specific integrated circuits or by interconnecting an appropriate network of conventional component circuits, as will be appreciated by those skilled in the electrical art(s).

In the context of this document, a "computer-readable medium" may be any media or means that can contain, store, communicate, propagate or transport the instructions for use by or in connection with an instruction execution system, apparatus, or device, such as a computer. A computer-readable medium may include a computer-readable storage medium that may be any media or means that can contain or store the instructions for use by or in connection with an instruction execution system, apparatus, or device, such as a computer. A computer readable medium can include any suitable medium that participates in providing instructions to a processor for execution. Such a medium can take many forms, including but not limited to, non-volatile media, volatile media, transmission media, and the like.

## Claims

1. A method for generating transformation matrices associated with upper and lower dental arches, the method comprising:
obtaining (100) a digital impression of said upper and lower dental arches, or a first digital impression of said upper dental arch and a second digital impression of said lower dental arch;
generating (102) a digital three-dimensional model comprising said upper and lower dental arches, or a first digital three-dimensional model comprising said upper dental arch and a second digital three-dimensional model comprising said lower dental arch based on said digital impression(s);
outputting (104) said digital three-dimensional model(s) to produce separate printed physical three-dimensional models of said upper and lower dental arches;
obtaining (106) scanning data associated with a desired target occlusion, said desired target occlusion being obtained based on a desired mutual position of the printed physical three-dimensional models of said upper and lower dental arches, the scanning data comprising only a portion of a complete buccal scan of the printed physical three-dimensional models of the upper and lower dental arches; and
determining (108) said transformation matrices of said upper and lower dental arches to said desired target occlusion based on said scanning data and said digital impression(s).

2. A method for generating transformation matrices associated with upper and lower dental arches, the method comprising:
obtaining (110) a digital impression of said upper and lower dental arches, or a first digital impression of said upper dental arch and a second digital impression of said lower dental arch, said digital impression(s) being based on printed physical three-dimensional models of said upper and lower dental arches, the printed physical three-dimensional models being prepared based on digital dental data;
obtaining (112) scanning data associated with a desired target occlusion, said desired target occlusion being obtained based on a desired mutual position of said printed physical three-dimensional models of said upper and lower dental arches, the scanning data comprising only a portion of a complete buccal scan of the printed physical three-dimensional models of the upper and lower dental arches; and
determining (114) said transformation matrices of said upper and lower dental arches to said desired target occlusion based on said scanning data and said digital impression(s).

3. A method of claim 1 or 2, wherein data associated with said digital impression(s) and/or scanning data are obtained from an intraoral scanning device.

4. A system for generating transformation matrices associated with upper and lower dental arches, the system comprising:
at least one processing unit (304);
at least one memory (306) connected to said at least one processing unit (304);
wherein said at least one memory (306) stores program instructions that, when executed by said at least one processing unit (304), cause said system (300) to:
obtain (100) a digital impression of upper and lower dental arches, or a first digital impression of said upper dental arch and a second digital impression of said lower dental arch;
generate (102) a digital three-dimensional model comprising said upper and lower dental arches, or a first digital three-dimensional model comprising said upper dental arch and a second digital three-dimensional model comprising said lower dental arch based on said digital impression(s);
output (104) said digital three-dimensional model(s) to produce separate printed physical three-dimensional models of said upper and lower dental arches;
obtain (106) scanning data associated with a desired target occlusion, said desired target occlusion being obtained based on a desired mutual position of said printed physical three-dimensional models of said upper and lower dental arches, the scanning data comprising only a portion of a complete buccal scan of the printed physical three-dimensional models of the upper and lower dental arches; and
determine (108) said transformation matrices of said upper and lower dental arches to said desired target occlusion based on said scanning data and said digital impression(s).

5. A system for generating transformation matrices associated with upper and lower dental arches, the system comprising:
at least one processing unit (304);
at least one memory (306) connected to said at least one processing unit (304);
wherein said at least one memory (306) stores program instructions that, when executed by said at least one processing unit (304), cause said system (300) to:
obtain (110) a digital impression of said upper and lower dental arches, or a first digital impression of said upper dental arch and a second digital impression of said lower dental arch, said digital impression(s) being based on printed physical three-dimensional models of said upper and lower dental arches, the printed physical three-dimensional models being prepared based on digital dental data;
obtain (112) scanning data associated with a desired target occlusion, said desired target occlusion being obtained based on a desired mutual position of said printed physical three-dimensional models of said upper and lower dental arches, the scanning data comprising only a portion of a complete buccal scan of the printed physical three-dimensional models of the upper and lower dental arches; and
determine (114) said transformation matrices of said upper and lower dental arches to said desired target occlusion based on said scanning data and said digital impression(s).

6. A system of claim 4 or 5, further comprising an intraoral scanning device configured to obtain the data associated with said digital impression(s) and/or scanning data.

7. A computer program comprising program code, which when executed by at least one processor (304), causes said at least one processor (304) to perform the method of any of claims 1 - 3.

8. A computer readable medium comprising program code, which when executed by at least one processor (304), causes said at least one processor (304) to perform the method of any of claims 1 - 3.

## Patentansprüche

1. Verfahren zur Erzeugung von Transformationsmatrizen in Verbindung mit oberen und unteren Zahnbögen, wobei das Verfahren umfasst:
Gewinnung (100) eines digitalen Abdrucks der oberen und unteren Zahnbögen, oder eines ersten digitalen Abdrucks des oberen Zahnbogens und eines zweiten digitalen Abdrucks des unteren Zahnbogens;
Erzeugen (102) eines digitalen dreidimensionalen Modells, das die oberen und unteren Zahnbögen umfasst, oder eines ersten digitalen dreidimensionalen Modells, das den oberen Zahnbogen umfasst und eines zweiten digitalen dreidimensionalen Modells, das den unteren Zahnbogen umfasst, basierend auf dem digitalen Abdruck/den digitalen Abdrücken;
Ausgeben (104) des/der digitalen dreidimensionalen Modells(e) zur Herstellung von separaten gedruckten physischen dreidimensionalen Modellen der oberen und unteren Zahnbögen;
Gewinnung (106) von Abtastdaten, die mit einer gewünschten Ziel-Okklusion verknüpft sind, wobei die gewünschte Ziel-Okklusion auf der Grundlage einer gewünschten gegenseitigen Position der gedruckten physischen dreidimensionalen Modelle der oberen und unteren Zahnbögen erhalten wird, wobei die Abtastdaten nur einen Teil einer vollständigen bukkalen Abtastung der gedruckten physischen dreidimensionalen Modelle der oberen und unteren Zahnbögen umfassen; und
Bestimmen (108) der Transformationsmatrizen der oberen und unteren Zahnbögen zu der gewünschten Zielokklusion auf der Grundlage der Abtastdaten und des digitalen Abdrucks/der digitalen Abdrucke.

2. Ein Verfahren zur Erzeugung von Transformationsmatrizen in Verbindung mit oberen und unteren Zahnbögen, wobei das Verfahren umfasst:
Gewinnung (110) eines digitalen Abdrucks der oberen und unteren Zahnbögen, oder einen ersten digitalen Abdrucks des oberen Zahnbogens und eines zweiten digitalen Abdrucks des unteren Zahnbogens, wobei der digitale Abdruck / die digitalen Abdrücke auf gedruckten physischen dreidimensionalen Modellen der oberen und unteren Zahnbögen basieren, wobei die gedruckten physischen dreidimensionalen Modelle auf der Grundlage digitaler Zahndaten erstellt werden;
Gewinnung (106) von Abtastdaten, die mit einer gewünschten Ziel-Okklusion verknüpft sind, wobei die gewünschte Ziel-Okklusion auf der Grundlage einer gewünschten gegenseitigen Position der gedruckten physikalischen dreidimensionalen Modelle der oberen und unteren Zahnbögen erhalten wird, wobei die Abtastdaten nur einen Teil einer vollständigen bukkalen Abtastung der gedruckten physischen dreidimensionalen Modelle der oberen und unteren Zahnbögen umfassen; und
Bestimmen (108) der Transformationsmatrizen der oberen und unteren Zahnbögen zu der gewünschten Zielokklusion auf der Grundlage der Abtastdaten und des digitalen Abdrucks/der digitalen Abdrucke.

3. Verfahren nach Anspruch 1 oder 2, wobei die mit dem digitalen Abdruck / den digitalen Abdrücken und/oder Abtastdaten verknüpften Daten von einer intraoralen Abtastvorrichtung gewonnen werden.

4. Ein System zur Erzeugung von Transformationsmatrizen in Verbindung mit oberen und unteren Zahnbögen, wobei das Verfahren umfasst:
mindestens eine Verarbeitungseinheit (304);
mindestens einen mit der mindestens einen Verarbeitungseinheit (304) verbundenen Speicher (306);
wobei der mindestens eine Speicher (306) Programmbefehle speichert, die bei Ausführung durch die mindestens eine Verarbeitungseinheit (304) das System veranlassen zur:
Gewinnung (100) eines digitalen Abdrucks der oberen und unteren Zahnbögen, oder eines ersten digitalen Abdruck des oberen Zahnbogens und eines zweiten digitalen Abdrucks des unteren Zahnbogens;
Erzeugung (102) eines digitalen dreidimensionalen Modells, das die oberen und unteren Zahnbögen umfasst, oder eines ersten digitalen dreidimensionalen Modells, das den oberen Zahnbogen umfasst, und eines zweiten digitalen dreidimensionalen Modells, das den unteren Zahnbogen umfasst, basierend auf dem digitalen Abdruck / den digitalen Abdrücken;
Ausgabe (104) des/der digitalen dreidimensionalen Modells/Modelle zur Herstellung separater gedruckter physischer dreidimensionaler Modelle der oberen und unteren Zahnbögen;
Gewinnung (106) von Abtastdaten, die mit einer gewünschten Ziel-Okklusion verknüpft sind, wobei die gewünschte Ziel-Okklusion auf der Grundlage einer gewünschten gegenseitigen Position der gedruckten physischen dreidimensionalen Modelle der oberen und unteren Zahnbögen erhalten wird, wobei die Abtastdaten nur einen Teil einer vollständigen bukkalen Abtastung der gedruckten physischen dreidimensionalen Modelle der oberen und unteren Zahnbögen umfassen; und
Bestimmen (108) der Transformationsmatrizen der oberen und unteren Zahnbögen zu der gewünschten Zielokklusion auf der Grundlage der Abtastdaten und des digitalen Abdrucks/der digitalen Abdrucke.

5. Ein System zur Erzeugung von Transformationsmatrizen in Verbindung mit oberen und unteren Zahnbögen, wobei das Verfahren umfasst:
mindestens eine Verarbeitungseinheit (304);
mindestens einen mit der mindestens einen Verarbeitungseinheit (304) verbundenen Speicher (306);
wobei der mindestens eine Speicher (306) Programmbefehle speichert, die bei Ausführung durch die mindestens eine Verarbeitungseinheit (304) das System veranlassen zur:
Gewinnung (100) eines digitalen Abdrucks der oberen und unteren Zahnbögen, oder eines ersten digitalen Abdruck des oberen Zahnbogens und eines zweiten digitalen Abdrucks des unteren Zahnbogens, wobei der digitale Abdruck / die digitalen Abdrücke auf gedruckten physischen dreidimensionalen Modellen der oberen und unteren Zahnbögen basieren, wobei die gedruckten physischen dreidimensionalen Modelle auf der Grundlage digitaler Zahndaten erstellt werden;
Gewinnung (112) von Abtastdaten, die mit einer gewünschten Ziel-Okklusion verknüpft sind, wobei die gewünschte Ziel-Okklusion auf der Grundlage einer gewünschten gegenseitigen Position der gedruckten physischen dreidimensionalen Modelle der oberen und unteren Zahnbögen erhalten wird, wobei die Abtastdaten nur einen Teil einer vollständigen bukkalen Abtastung der gedruckten physischen dreidimensionalen Modelle der oberen und unteren Zahnbögen umfassen; und
Bestimmen (114) der Transformationsmatrizen der oberen und unteren Zahnbögen zu der gewünschten Zielokklusion auf der Grundlage der Abtastdaten und des digitalen Abdrucks/der digitalen Abdrucke.

6. Ein System nach Anspruch 4 oder 5, das ferner eine intraorale Abtastvorrichtung umfasst, die so konfiguriert ist, dass sie die mit dem digitalen Abdruck / den digitalen Abdrücken und/oder den Abtastdaten verknüpften Daten gewinnt.

7. Ein Computerprogramm mit Programmcode, der bei Ausführung von mindestens einem Prozessor (304) den mindestens einen Prozessor (304) veranlasst, das Verfahren nach einem der Ansprüche 1 bis 3 durchzuführen.

8. Ein computerlesbares Medium mit Programmcode, das bei Ausführung durch mindestens einen Prozessor (304) den mindestens einen Prozessor (304) veranlasst, das Verfahren nach einem der Ansprüche 1 bis 3 durchzuführen.

## Revendications

1. Procédé pour générer des matrices de transformation associées à des arcades dentaires supérieure et inférieure, le procédé comprenant :
l'obtention (100) d'une empreinte numérique desdites arcades dentaires supérieure et inférieure ou d'une première empreinte numérique de ladite arcade dentaire supérieure et d'une seconde empreinte numérique de ladite arcade dentaire inférieure ;
la génération (102) d'un modèle numérique tridimensionnel comprenant lesdites arcades dentaires supérieure et inférieure ou d'un premier modèle numérique tridimensionnel comprenant ladite arcade dentaire supérieure et d'un second modèle numérique tridimensionnel comprenant ladite arcade dentaire inférieure sur la base de ladite ou desdites empreintes numériques ;
la fourniture en sortie (104) dudit ou desdits modèles numériques tridimensionnels pour produire des modèles physiques tridimensionnels imprimés séparés desdites arcades dentaires supérieure et inférieure ;
l'obtention (106) de données de balayage associées à une occlusion cible souhaitée, ladite occlusion cible souhaitée étant obtenue sur la base d'une position mutuelle souhaitée des modèles physiques tridimensionnels imprimés desdites arcades dentaires supérieure et inférieure, les données de balayage comprenant seulement une partie d'un balayage buccal complet des modèles physiques tridimensionnels imprimés des arcades dentaires supérieure et inférieure ; et
la détermination (108) desdites matrices de transformation desdites arcades dentaires supérieure et inférieure jusqu'à ladite occlusion cible souhaitée sur la base desdites données de balayage et de ladite ou desdites empreintes numériques.

2. Procédé pour générer des matrices de transformation associées à des arcades dentaires supérieure et inférieure, le procédé comprenant :
l'obtention (110) d'une empreinte numérique desdites arcades dentaires supérieure et inférieure ou d'une première empreinte numérique de ladite arcade dentaire supérieure et d'une seconde empreinte numérique de ladite arcade dentaire inférieure, ladite ou lesdites empreintes numériques étant basées sur des modèles physiques tridimensionnels imprimés desdites arcades dentaires supérieure et inférieure, les modèles physiques tridimensionnels imprimés étant préparés sur la base de données dentaires numériques ;
l'obtention (112) de données de balayage associées à une occlusion cible souhaitée, ladite occlusion cible souhaitée étant obtenue sur la base d'une position mutuelle souhaitée desdits modèles physiques tridimensionnels imprimés desdites arcades dentaires supérieure et inférieure, les données de balayage comprenant seulement une partie d'un balayage buccal complet des modèles physiques tridimensionnels imprimés des arcades dentaires supérieure et inférieure ; et
la détermination (114) desdites matrices de transformation desdites arcades dentaires supérieure et inférieure jusqu'à ladite occlusion cible souhaitée sur la base desdites données de balayage et de ladite ou desdites empreintes numériques.

3. Procédé selon la revendication 1 ou 2, dans lequel des données associées à ladite ou auxdites empreintes numériques et/ou des données de balayage sont obtenues à partir d'un dispositif de balayage intra-oral.

4. Système pour générer des matrices de transformation associées aux arcades dentaires supérieure et inférieure, le système comprenant :
au moins une unité de traitement (304) ;
au moins une mémoire (306) raccordée à ladite au moins une unité de traitement (304) ;
dans lequel ladite la au moins une mémoire (306) stocke des instructions de programme qui, lorsqu'elles sont exécutées par la ladite au moins une unité de traitement (304), amènent ledit système (300) à :
obtenir (100) une empreinte numérique d'arcades dentaires supérieure et inférieure ou une première empreinte numérique de ladite arcade dentaire supérieure et une seconde empreinte numérique de ladite arcade dentaire inférieure ;
générer (102) un modèle numérique tridimensionnel comprenant lesdites arcades dentaires supérieure et inférieure ou un premier modèle numérique tridimensionnel comprenant ladite arcade dentaire supérieure et un second modèle numérique tridimensionnel comprenant ladite arcade dentaire inférieure sur la base de ladite ou desdites empreintes numériques ;
délivrer (104) ledit ou lesdits modèles numériques tridimensionnels pour produire des modèles physiques tridimensionnels imprimés séparés desdites arcades dentaires supérieure et inférieure ;
obtenir (106) des données de balayage associées à une occlusion cible souhaitée, ladite occlusion cible souhaitée étant obtenue sur la base d'une position mutuelle souhaitée desdits modèles physiques tridimensionnels imprimés desdites arcades dentaires supérieure et inférieure, les données de balayage comprenant seulement une partie d'un balayage buccal complet des modèles physiques tridimensionnels imprimés des arcades dentaires supérieure et inférieure ; et
déterminer (108) lesdites matrices de transformation desdites arcades dentaires supérieure et inférieure vers ladite occlusion cible souhaitée sur la base desdites données de balayage et de ladite ou desdites empreintes numériques.

5. Système pour générer des matrices de transformation associées aux arcades dentaires supérieure et inférieure, le système comprenant :
au moins une unité de traitement (304) ;
au moins une mémoire (306) raccordée à ladite au moins une unité de traitement (304) ;
dans lequel ladite au moins une mémoire (306) stocke des instructions de programme qui, lorsqu'elles sont exécutées par ladite au moins une unité de traitement (304), amènent ledit système (300) à :
obtenir (110) une empreinte numérique desdites arcades dentaires supérieure et inférieure ou une première empreinte numérique de ladite arcade dentaire supérieure et une seconde empreinte numérique de ladite arcade dentaire inférieure, ladite ou lesdites empreintes numériques étant basées sur des modèles physiques tridimensionnels imprimés desdites arcades dentaires supérieure et inférieure, les modèles physiques tridimensionnels imprimés étant préparés sur la base de données dentaires numériques ;
obtenir (112) des données de balayage associées à une occlusion cible souhaitée, ladite occlusion cible souhaitée étant obtenue sur la base d'une position mutuelle souhaitée desdits modèles physiques tridimensionnels imprimés desdites arcades dentaires supérieure et inférieure, les données de balayage comprenant seulement une partie d'un balayage buccal complet des modèles physiques tridimensionnels imprimés des arcades dentaires supérieure et inférieure ; et
déterminer (114) lesdites matrices de transformation desdites arcades dentaires supérieure et inférieure vers ladite occlusion cible souhaitée sur la base desdites données de balayage et de ladite ou desdites empreintes numériques.

6. Système selon la revendication 4 ou 5, comprenant en outre un dispositif de balayage intra-oral configuré pour obtenir les données associées à ladite ou auxdites empreintes numériques et/ou des données de balayage.

7. Programme informatique comprenant un code de programme qui, lorsqu'il est exécuté par au moins un processeur (304), amène ledit au moins un processeur (304) à réaliser le procédé selon l'une quelconque des revendications 1-3.

8. Support lisible par ordinateur comprenant un code de programme qui, lorsqu'il est exécuté par au moins un processeur (304), amène ledit au moins un processeur (304) à réaliser le procédé selon l'une quelconque des revendications 1-3.
